# EUROPEAN PATENT APPLICATION

(11) **EP 2 777 748 A1**
(43) Date of publication of application: **17.09.2014**
(21) Application number: 13290064.8
(22) Date of filing: 15.03.2013
(51) Int. Cl.: A61M 25/06, A61M 25/01, A61M 25/00

(54) **Catheter-access sheath assembly and a security guide next to the access sheath**

(71) Applicant: Coloplast A/S, 3050 Humlebæk (DK)
(72) Inventor: Lafitte, Mathieu, 24200 Sainte Nathalene (FR); Pascal, Laurent, 24200 Sarlat La Caneda (FR)

(57) **Abstract**

The present invention concerns a catheter-access sheath assembly (20) comprising a catheter (2) and an access sheath (1) characterized in that the catheter comprises at least a groove (23) extending between an outer hole (12) and an inner hole (11) having a greater width than the width of the security guide (3), in that the access sheath (1) comprises an opening (21) extending longitudinally from a distal end of the access sheath (1), in that the access sheath (1) is rotatable around the catheter (2) between an open position in which the opening (21) of the access sheath (1) at least partially coincides with the groove (23) of the catheter and enables the release of the security guide (3), and a closed position in which the access sheath (1) covers the groove (23) of the catheter (2) and enables to maintain the security guide within the groove (23).

## Description

### TECHNICAL FIELD

The present invention concerns a catheter-access sheath assembly comprising a catheter and an access sheath, said assembly being intended for positioning the access sheath in a working position and a security guide next to the access sheath, wherein the access sheath is adapted to be threaded onto the catheter.

The present invention also concerns a method for positioning an access sheath and a security guide next to the access sheath, said method using a catheter-access sheath assembly comprising a catheter, said catheter comprising a groove and said access sheath comprising an opening extending longitudinally from a distal end of the access sheath, said access sheath being rotatable around the catheter between an open position in which the opening of the access sheath at least partially coincides with the groove of the catheter and enables the release of the security guide, and a closed position in which the access sheath covers the groove of the catheter and enables to maintain the security guide within the groove.

That kind of catheter-access sheath assembly is used by surgeons to enable them making an intervention with a tool in areas within the human body which can be difficult to access. These assemblies are used, among others, in urology.

That is in this domain that the problem that has given rise to the invention of the present application has arisen. However, the Applicant does not intend to limit the scope of their rights to this particular application.

### BACKGROUND ART

When a surgeon wants to access to a kidney, if the surgical intervention is dismissed, he has, starting from the natural routes of entry, to go up inside the urethra, pass the bladder and then, beyond the urethral meatus, go up inside the urethra to reach the kidney.

Most often, the positioning of an access sheath was performed by a quite restrictive method. With the aid of a cystoscope, which is a tubular element carrying an optical fiber - it is a bladder endoscope -, previously introduced inside the bladder, a first radio-opaque guide is driven up to the bladder. Then, with the aid of the cystoscope, the urethral meatus is targeted at in order to introduce the guide inside the urethra. The guide is a generally sheathed nickel, titanium and/or stainless steel alloy lead.

After the setting up of this first guide, a radio-opaque double channel urethral probe or catheter is engaged by one of its both channels onto the guide and it is driven up to the urethra. Through the other channel of the urethral probe, a second guide is introduced till the urethra. Then the urethral probe is removed to let behind only both guides, a working guide and a security guide, this latter being fixed onto the patient.

During these first steps, the radio-opaque components have been visualized to check their positions.

The access sheath being threaded into a dilator projecting forwards outside the sheath, the access sheath is engaged into the working guide through the dilator channel, then the sheath end is driven up to a position between the bladder and the kidney, but nearer the bladder. The dilator and the working guide are removed to let behind in place only the access sheath, and near it, the security guide that can be used in case of difficulty.

The course of all these steps of positioning the access sheath highlights the fundamental problem of the invention: reducing the number of positioning steps in order to save time and reduce the risks for the patient and limiting the number of necessary components (working and security guides, urethral probe for positioning the security guide and the dilator).

Thus, the invention of the present application relates to a catheter-access sheath assembly enabling an enhanced positioning of an access sheath and a security guide next to the sheath, comprising the steps of positioning a guide and engaging, driving up and setting up a catheter onto the guide. The access sheath being mounted onto the catheter also plays a role of dilator, after setting up of the catheter on the guide, the access sheath is driven up onto the catheter in order to set it up, have the guide exit from the catheter and lay it next to the sheath and the catheter-dilator is removed.

According to this method, only one guide is used. It is first used as a working guide before being turned into a security guide. The reduction of the number of components is an important advantage as well as the number of placing steps thanks to the double function of the urethral catheter i) for positioning the security guide and ii) as a dilator.

Preferably, the catheter comprises a setting up channel and a channel for placing a security guide. For setting up the catheter, the security guide is engaged by the inner end of the security channel before driving up the access sheath onto the catheter.

The inner end or distal end of the channel means its end intended, after setting up, to be in an area of the intervention or in the nearest from this area. Conversely, the outer end or proximal end of the channel is that which will be at the entry of the natural route of the body or the nearest one from this natural entry.

The positioning method used with the catheter-access sheath assembly of the invention can also resort to two different guides, a working guide and a security guide.

The catheter comprising a setting up channel and a channel for positioning a security guide, for setting up the catheter, a working guide is first engaged through the inner end of the catheter setting up channel. Then, after setting up, a security guide is introduced inside the security channel of the catheter through its outer end. The access sheath is then pulled up on the catheter to have the security guide exit from the catheter and extending it next to the sheath. The catheter and the working guide are then removed. This two guide positioning method should be better suitable to less experimented operators.

The patent application WO 2009/127216 describes a catheter-access sheath assembly enabling to simplify the method for positioning an access sheath to access for example, but not exclusively, to a kidney. The catheter-access sheath assembly according to this application comprises means for positioning a security guide next to the sheath. In this invention, the catheter comprises two longitudinal channels, one of them being used as a security channel for receiving a security guide. The catheter further comprises an outer hole and an inner hole, the wall of the catheter extending between both holes of the security channel being arranged to open under the action of a peel strength directed from inside to outside of the channel.

Although this catheter-access sheath assembly works perfectly well, it has some limitations. In particular, the wall of the security channel between both holes forms lips. These lips are arranged to be closed in the absence of strength applied to them. When the catheter-access sheath assembly is manipulated to remove the catheter and to put the security guide in place, strength is applied on the lips. This strength has the effect of opening the lips and enabling the security guide to exit the security channel of the catheter. This implies several constraints. A first constraint concerns the material. The material of the catheter must be sufficiently flexible to enable the lips to open and close, and sufficiently stiff to maintain the security guide within the security channel.

A second constraint concerns the relative size of the security guide and of the lips. Of course, the lips, when fully open, must be broader than the diameter of the security guide. When closed, the lips must be either fully closed or at least narrower than the diameter of the security guide. This can mean that, for one type of catheter, having one dimension of lips, it is not possible to use several types of security guide having very different diameters.

The present invention seeks to solve this problem by proposing a catheter-access sheath assembly in which several types of security guides can be used and in which several types of material can also be used.

### DISCLOSURE OF INVENTION

The object of the invention is achieved by a catheter-access sheath assembly such as described in the preamble and **characterized in that** the catheter comprises at least a groove extending between an outer hole and an inner hole, said groove having a greater width than the width of the security guide, in that the access sheath comprises an opening extending longitudinally from a distal end of the access sheath, in that the access sheath is rotatable around the catheter between an open position in which the opening of the access sheath at least partially coincides with the groove of the catheter and enables the release of the security guide, and a closed position in which the access sheath covers the groove of the catheter and enables to maintain the security guide within the groove.

The object of the invention is also achieved by a method as described in the preamble and **characterized in that** it comprises the steps of :
- positioning the security guide in a place where the security guide will be used;
- positioning a portion of the security guide in the groove of the catheter;
- placing the access sheath onto the catheter in a closed position in such a way that said portion of the security guide is maintained between the catheter and the access sheath;
- driving the access sheath and the catheter along the security guide to the place where the access sheath will be used;
- rotating the access sheath with regard to the catheter to reach the open position in which the security guide exits the groove of the catheter;
- removing the catheter.

According to the present invention, the catheter-access sheath assembly can be made of a great range of different materials, without the necessity of being flexible. This opens the door to materials that can be easier to manufacture, to sterilize or to store for example.

Moreover, as the invention does not rely on flexibility of material, the same catheter and access sheath can accommodate a great variety of diameters for the security guide. This also opens the door to different applications.

The shape of the groove and opening on the catheter and access sheath can be made to facilitate the manipulation of the assembly. In particular, it is quite easy to imagine a shape of groove that enables easy introduction of the security guide and a shape of opening that enables an efficient maintenance of the guide It is for example possible to introduce the security guide when the assembly is in an open position and to simply turn the access sheath to bring the assembly in a closed position and to maintain the security guide.

### BRIEF DESCRIPTION OF DRAWINGS

The present invention and its advantages will be better understood with reference to the enclosed drawings and to the detailed description of a specific embodiment, in which :

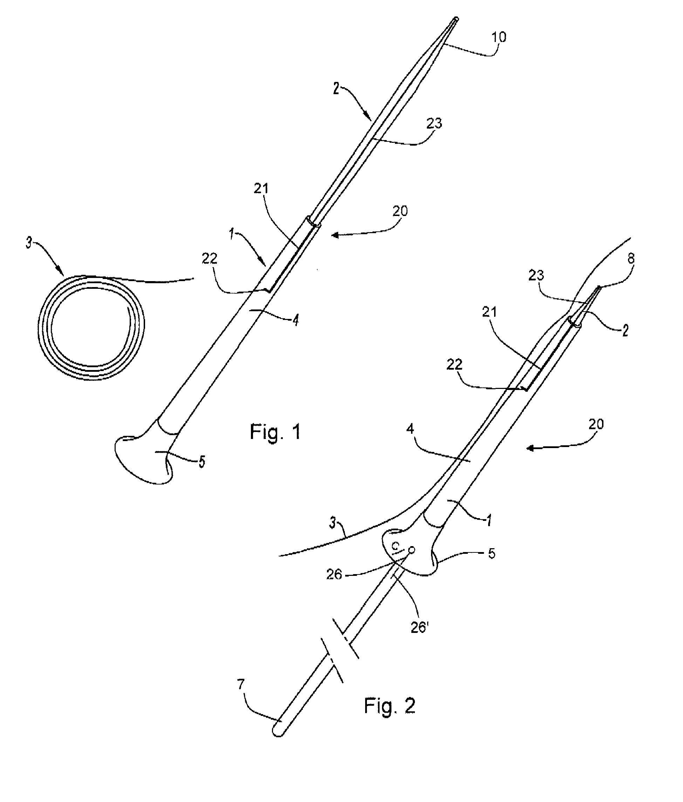

- Fig. 1 is a perspective view of an catheter-access sheath assembly according to the present invention, as well as a security guide used with this assembly;
- Fig 2 is a perspective view of the assembly of Fig. 1, the access sheath being driven up onto the catheter and the security guide extending next to the sheath;

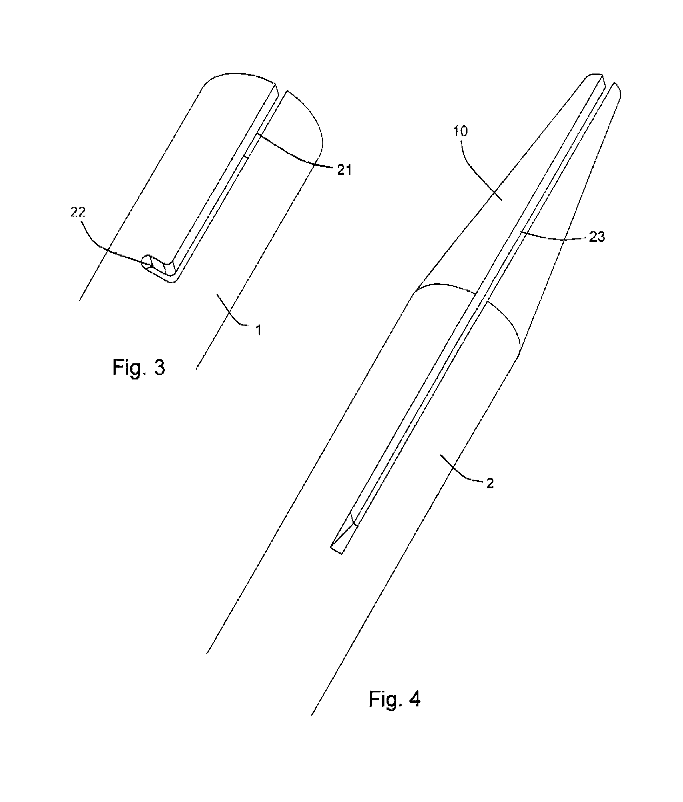

- Fig. 3 is a perspective view of the access sheath according to the invention;
- Fig. 4 is a perspective view of the catheter according to the invention;
- Fig. 5 is a side view of the catheter-access sheath assembly in an open position;
- Fig. 6 is a cross section view along the line A-A of the assembly of Fig. 5;
- Fig. 7a shows a first embodiment of a cross section along the line B-B of the assembly of Fig. 5;
- Fig. 7b shows a second embodiment of a cross section along the line B-B of the assembly of Fig. 5;
- Fig. 8 is a side view of the catheter-access sheath assembly in an closed position; and
- Fig. 9 is a cross section view of the assembly of Fig. 8.

### BEST MODE FOR CARRYING OUT THE INVENTION

According to the present invention, the catheter-access sheath assembly 20 comprises an access sheath 1 and a catheter 2. This assembly is foreseen to cooperate with a security guide 3.

The access sheath 1 is provided for accessing to a place to be operated, for example the urinary tract. The access sheath 1 is formed of a reinforced tube 4 that is flexible but resistant to collapse, compression and plication. The access sheath further comprises a bucket 5 on its proximal end provided for an easy introduction of instruments and tools.

According to the present invention, the access sheath 1 comprises a longitudinal opening 21 extending from the distal end of the access sheath. This opening 21 is substantially straight over a given length of the access sheath and ends by a short bend 22 whose function is explained below.

According to a first embodiment, the catheter 2 is a flexible tube comprising two separate longitudinal hollow channels 6₁ and 6₂ both extending between a proximal end 7 and a distal end 8 The distal end 8 is slightly frustoconical in direction of a tip 10. According to a second embodiment, the catheter 2 comprises one hollow channel 6₁ extending from the proximal end to the distal end. In both embodiments, the catheter further contains a groove 23 extending from the tip 10 of the catheter or close to it over a given length in direction of the proximal end 7 of the catheter.

In the first embodiment, i.e. in the case where the catheter 2 comprises two hollow channels, the groove 23 may communicate with one of the hollow channels 6₂. In the second embodiment, i.e. in the case where the catheter 2 comprises only one hollow channel 6₁, the groove 23 does preferably not communicate with the hollow channel.

The groove 23 has a width that is sufficiently large to enable an easy insertion and removal of a security guide 3.

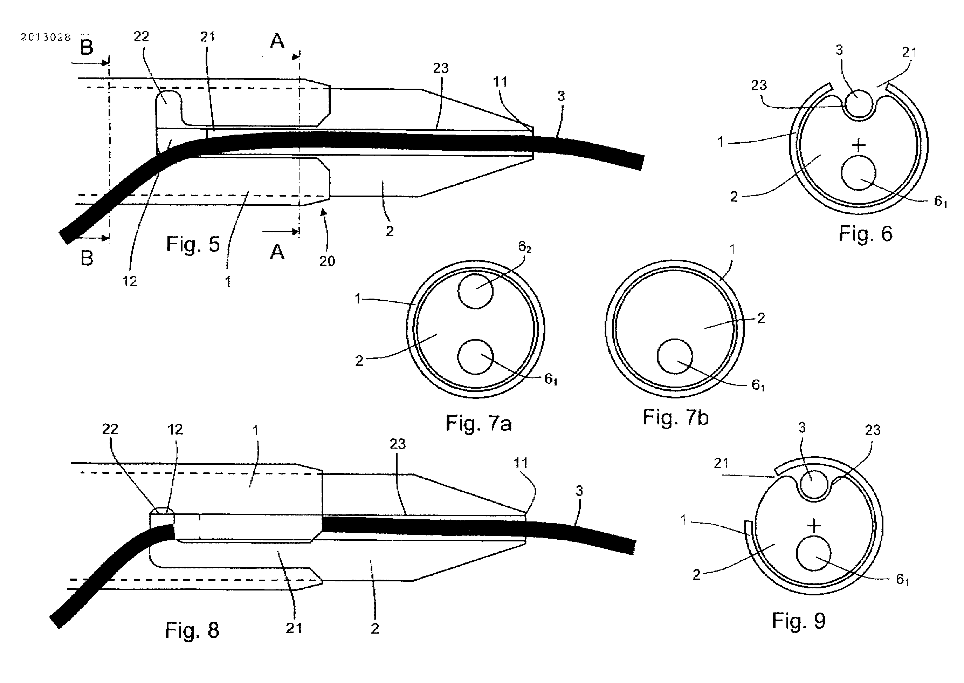

The access sheath 1 can move on the catheter 2 along a longitudinal axis. It can further be rotated on the catheter 2 around a longitudinal axis between at least two positions. In a first position, called open position and illustrated by Fig. 5 and 6, the opening 21 of the access sheath 1 at least partially coincides with the groove 23 of the catheter and enables the release of the security guide 3. In a second position, called closed position and illustrated by Fig. 8 and 9, the access sheath 1 covers the groove 23 of the catheter 2 and enables to maintain the security guide 3 within the groove 23.

In the closed position, the bend 22 of the opening 21 of the access sheath coincides with the groove 23 of the catheter 2. Thus, the security guide 3 can enter the groove 23 by an inner hole 11 and exits the groove by an outer hole 12 formed by the superposition of the groove 23 and the bend 22.

According to a preferred embodiment, the catheter 2 and the access sheath 1 comprise a marking 26, 26' indicating the open position, the closed position or both. The marking could also indicate a direction of rotation to move the access sheath 1 from the open to the closed position or vice versa.

According to a variant, the catheter 2 or the access sheath 1 may comprise a protrusion (not represented) cooperating with a recess (not represented) in the corresponding part, i.e. either the access sheath 1 or the catheter 2. The protrusion and recess are intended to limit the rotation of the access sheath 1 compared to the catheter 2 to reach the open and closed position.

When the catheter-access sheath assembly 20 must be used, the security guide 3 is introduced in the place where the operation will take place. For example, the catheter can be positioned up to the urethra, after introduction through the natural entry route, using a cystoscope.

Once the security guide 3 is positioned, the catheter 2 is engaged onto the guide 3. More specifically, the security guide 3 is positioned into the groove 23 of the catheter. The access sheath 1 is placed around the catheter in such a way that the opening 21 of the access sheath 1 coincides with the groove 23 of the catheter 2. The access sheath is then rotated in order to imprison the security guide 3 in the groove 23 of the catheter, the access sheath preventing the security guide from leaving this groove. The security guide 3 exits the access sheath through the bend 22 of the opening 21, which forms the outer hole 12.

The security guide 3 can be used as a glider to position the catheter-access sheath assembly 20 to operate in a conventional way. When the catheter-access sheath assembly has to be removed, while keeping the security guide in place, the access sheath 1 is rotated with regard to the catheter 2. The access sheath is rotated until the longitudinal part of the opening 21 coincides with the groove 23 of the catheter. It should be noted that the rotation in closed or open position may be limited by the protrusion and recess on the access sheath and on the catheter. However, it may also be limited only by the security guide 3. In the closed position, the security guide is squeezed between the groove 23 of the catheter and the bend 22 of the opening 21 of the access sheath. In the open position, the security guide 3 is squeezed between the groove 23 of the catheter 2 and the part of the opening 21 opposite to the bend 22.

When the groove 23 coincides with the longitudinal part of the opening 21, the security guide 3 may exit the groove and it will thus be placed next to the catheter-access sheath assembly.

The present invention works the same way whether the catheter has one or two hollow channels 6₁, 6₂. In case it has two channels, one of them ending by the groove 23, the security guide 3 can be put in place as describe above. One channel is dedicated to the security guide and the other channel can be used for other tasks. In case the catheter has only one channel, it does not interfere with the groove 23.

Thank to this invention, the material used for manufacturing the catheter does not have to be elastic and the range of usable material is greater. Moreover, the width of the groove and opening does not have to be closely adapted to the diameter of the security guide. The only important thing is that the security guide can be maintained in the groove in closed position and removed from the groove and opening in open position.

## Claims

1. Catheter-access sheath assembly (20) comprising a catheter (2) and an access sheath (1), said assembly being intended for positioning the access sheath (1) in a working position and a security guide (3) next to the access sheath (1), wherein the access sheath (1) is adapted to be threaded onto the catheter (2), said assembly being **characterized in that** the catheter (2) comprises at least a groove (23) extending between an outer hole (12) and an inner hole (11), said groove (23) having a greater width than the width of the security guide (3), **in that** the access sheath (1) comprises an opening (21) extending longitudinally from a distal end of the access sheath (1), **in that** the access sheath (1) is rotatable around the catheter (2) between an open position in which the opening (21) of the access sheath (1) at least partially coincides with the groove (23) of the catheter and enables the release of the security guide (3), and a closed position in which the access sheath (1) covers the groove (23) of the catheter (2) and enables to maintain the security guide (3) within the groove (23).

2. Catheter-access sheath assembly according to claim 1, **characterized in that** the opening (21) of the access sheath (1) comprises a bend (22) at a proximal end, and **in that** said bend (22) coincides with the groove (23) in the closed position.

3. Catheter-access sheath assembly according to claim 1, **characterized in that** the catheter (2) and the access sheath (1) comprise at least one marking (26, 26') indicating the open position, said marking being visible while the catheter-access sheath assembly is being used

4. Catheter-access sheath assembly according to any of claims 1 to 3, **characterized in that** the catheter (2) and the access sheath (1) comprise at least one marking (26, 26') indicating the closed position said marking being visible while the catheter-access sheath assembly is being used.

5. Catheter-access sheath assembly according to claim 1, **characterized in that** the access sheath (1) comprises at least one marking (26, 26') indicating the relative rotation direction of the access sheath (1) and of the catheter (2) to reach the open or closed position said marking being visible while the catheter-access sheath assembly is being used.

6. Catheter-access sheath assembly according to claim 1, **characterized in that** the catheter (2) comprises a protrusion cooperating with a recess in the access sheath (1), the protrusion and recess limiting the rotation of the access sheath (1) compared to the catheter (2), to reach the open and closed position.

7. Catheter-access sheath assembly according to claim 1, **characterized in that** the inner hole (11) of the catheter (2) coincides with the distal end of the catheter (2)

8. Catheter-access sheath assembly according to claim 1, **characterized in that** the catheter (2) comprises two hollow channels (6₁, 6₂), one of said hollow channels (6₂) communicating with the groove (23) of the catheter.

9. Catheter-access sheath assembly according to claim 1, **characterized in that** the catheter (2) comprises one hollow channel (6₁) which do not communicate with the groove (23) of the catheter.

10. Method for positioning an access sheath (1) and a security guide (3) next to the access sheath (1), said method using a catheter-access sheath assembly (20) comprising a catheter (2), said catheter (2) comprising a groove (23) and said access sheath (1) comprising an opening (21) extending longitudinally from a distal end of the access sheath (1), said access sheath (1) being rotatable around the catheter (2) between an open position in which the opening (21) of the access sheath (1) at least partially coincides with the groove (23) of the catheter and enables the release of the security guide (3), and a closed position in which the access sheath (1) covers the groove (23) of the catheter (2) and enables to maintain the security guide (3) within the groove (23), said method being **characterized in that** it comprises the steps of :
- positioning the security guide (3) in a place where the security guide (3) will be used;
- positioning a portion of the security guide (3) in the groove (23) of the catheter (2);
- placing the access sheath (1) onto the catheter (2) in a closed position in such a way that said portion of the security guide (3) is maintained between the catheter (2) and the access sheath (1);
- driving the access sheath (1) and the catheter (2) along the security guide (3) to the place where the access sheath (1) will be used;
- rotating the access sheath (1) with regard to the catheter (2) to reach the open position in which the security guide (3) exits the groove (23) of the catheter (2);
- removing the catheter (2).
